# EUROPÄISCHE PATENTSCHRIFT

(11) **EP 1 754 508 B1**
(45) Veröffentlichungstag und Bekanntmachung des Hinweises auf die Patenterteilung: **29.09.2010**
(21) Anmeldenummer: 06014989.5
(22) Anmeldetag: 19.07.2006
(51) Int. Cl.: A61N 1/05

(54) **Medizinische Elektrodenvorrichtung, insbesondere implantierbare kardiologische Elektrodenvorrichtung**
Medical electrode device, in particular implantable cardiac electrode device
Electrode médicale, en particulier électrode cardiaque implantable

(30) Priorität: 18.08.2005 DE 102005039039
(43) Veröffentlichungstag der Anmeldung: 21.02.2007
(73) Patentinhaber: Biotronik CRM Patent AG, 6341 Baar (CH)
(72) Erfinder: Junge, Agur, 8424 Embrach (CH)
(74) Vertreter: Lindner-Vogt, Karin L.

(56) Entgegenhaltungen:
- EP-A- 0 786 828
- DE-C1- 3 150 568
- US-A- 2 801 396
- US-A- 4 711 027

## Beschreibung

Die Erfindung betrifft eine medizinische Elektrodenvorrichtung und insbesondere eine implantierbare kardiologische Elektrodenvorrichtung mit den im Oberbegriff des Patentanspruches 1 angegebenen Merkmalen.

Derartige Elektrodenvorrichtungen sind beispielsweise in Form von Herz-Elektrodenkathetern seit langem in unterschiedlichsten Ausführungsformen bekannt. Sie haben einen lang gestreckten, schlauchartigen Elektrodenkörper aus einem medizinisch verträglichen Isoliermaterial und mindestens eine Elektrode am Elektrodenkörper zur Messung kardiologischer Reizpotentiale und/oder zur Abgabe von therapeutisch wirksamen elektrischen Signalen gemeinsam. Dokument US-A-4 711 027 offenbart den Oberbegriff des Anspruchs 1.

Die vorliegende Erfindung bezieht sich dabei auf um die Längsachse der Elektrodenvorrichtung rotationssymmetrische Elektroden, also insbesondere auf Ringelektroden oder auch die in der Regel kappenförmigen Tip-Elektroden am distalen Ende des Elektrodenkörpers.

Zur elektrischen Anbindung der Elektrode verläuft im Elektrodenkörper eine elektrische Leitung, die in der Regel gewendelt ist. Wie üblich weist diese Leitung einen elektrisch leitenden Kern und eine Isolierung auf, wobei der Kern mit der Elektrode elektrisch kontaktverbunden ist.

Zur Herstellung dieser elektrischen Kontaktverbindung ist es üblich, den Kern der aufgrund der Dimensionen solcher Elektrodenvorrichtungen sehr filigranen Leitungen mit Hilfe eines Skalpells abzuisolieren, um anschließend die Kontaktverbindung mit der Elektrode auf mechanischem Wege oder beispielsweise durch Löten herzustellen. Auch ein Salzstrahlen zum Entfernen der Isolierung wird praktiziert, ist allerdings ebenso ein äußerst aufwändiger Herstellungsschritt.

Davon ausgehend liegt der Erfindung die Aufgabe zugrunde, eine medizinische Elektrodenvorrichtung im Bereich der elektrischen Kontaktverbindung zwischen Elektrode und Leitung so zu verbessern, dass eine dauerstabile, sichere Kontaktierung auf einfachem Wege und insbesondere ohne einen Abisolierschritt gewährleistet ist.

Diese Aufgabe wird durch die im Kennzeichnungsteil des Anspruches 1 angegebenen Merkmale gelöst. Demnach ist eine rein mechanische Kontaktverbindung zwischen Elektrode und Leitung vorgesehen, die auf einer Stützhülse im Elektrodenkörper aufbaut. Auf dieser ist der zu kontaktierende Leitungsabschnitt der Leitung in nicht abisoliertem Zustand aufgewickelt, worauf die Elektrode unter Umfassen dieses Leitungsabschnittes sitzt. Dabei weist die Elektrode eine Schneidzone an ihrer Innenseite auf, mit der sie unter Durchbrechen der Isolierung der Leitung mit deren Kern in elektrischem Kontakt steht.

Für die Herstellung des Kontaktes kann also ein Abisolieren der Leitung völlig unterbleiben. Vielmehr wird die Isolierung beim Aufbringen der Elektrode auf die Stützhülse mit dem zu kontaktierenden Leitungsabschnitt durch die Einwirkung der Schneidzone überwunden. Bevorzugtermaßen kann dies konstruktiv besonders elegant dadurch realisiert werden, dass als Schneidzone an der Elektrode ein Innengewinde vorgesehen ist, mit dem die Elektrode auf den zu kontaktierenden Leitungsabschnitt aufgeschraubt wird. Der Aufschraubvorgang dient dabei nicht nur der elektrischen Kontaktierung, sondern stellt gleichzeitig die mechanische Lagerung und Festlegung der Elektrode auf der Stützhülse mit der dazwischen liegenden Leitung dar.

Gemäß einer weiteren bevorzugten Ausführungsform kooperiert das Innengewinde der Elektrode mit einem Außengewinde auf der Stützhülse, so dass eine sauber geführte, stabile und passgenaue Halterung der Elektrode auf der Stützhülse bei gleichzeitiger Herstellung der elektrischen Kontaktverbindung erreicht wird. Dabei ist ferner von Vorteil, dass bei einem kurzen Zurückdrehen der Elektrode gegen die Einschraubdrehrichtung der Drahtkern des kontaktieren Leitungsabschnittes einen Aufspann- oder -spreizeffekt zeigt, der ein weiteres Zurückdrehen der Elektrode und damit ein Lösen von der Stützhülse unterbindet. Die Elektrode ist damit dauerhaft auf der Stützhülse festgelegt.

In einer weiteren bevorzugten Ausführungsform sind als Montagehilfen Anschläge an Stützhülse und Elektrode vorgesehen, die die Einschraubtiefe zwischen diesen beiden Teilen begrenzen. Insoweit wird eine Fehlmontage wirkungsvoll verhindert.

Weitere vorteilhafte Ausführungsformen beziehen sich insbesondere auf Elektrodenvorrichtungen, bei denen die elektrische Kontaktierung der Elektroden am distalen Ende mit den bereits eingangs erwähnten wendelförmigen Mehrfachleitungsbündeln erfolgt. Demnach weist die Stützhülse mindestens einen Längsschlitz auf, durch den ein den zu kontaktierenden Leitungsabschnitt bildendes, aus dem Mehrfachleitungsbündel ausgewendeltes Teilbündel nach außen durch die Stützhülse zum Aufwickeln auf jeder Außenseite herausgeführt ist. Über den gleichen oder einen anderen Längsschlitz kann dann die herausgeführte Leitung auf das restliche Mehrfachleitungsbündel zurückgeführt und dort weiter gewickelt werden. Bevorzugtermaßen wird dabei zur weiteren Isolierung der einzelnen Bündel untereinander, die in der Regel verschiedene Elektroden kontaktieren, eine Isolierlage zwischengesetzt sein. Damit wird die elektrische Trennung zwischen den verschiedenen, von den Teilbündeln des Mehrfachleitungsbündels versorgten Polen zusammen mit der mechanischen Abdichtung des Elektrodeninnenraumes im Bereich der durch die aufgesetzte Elektrode entstehenden Fugen zum Elektrodenkörper hin verbessert.

Weitere Merkmale, Einzelheiten und Vorteile der Erfindung sind der nachfolgenden Beschreibung entnehmbar, in der Ausführungsbeispiele des Erfindungsgegenstandes anhand der beigefügten Zeichnungen näher erläutert werden. Es zeigen:
- Fig. 1: einen stark vergrößerten, schematischen, teilweisen Axialschnitt einer Elektrodenvorrichtung in einer ersten Ausführungsform,
- Fig. 2: eine gegenüber dem Schnitt von Fig. 1 um 90° um die Rotationsachse gedrehte schematische Ansicht der Elektrodenvorrichtung in einem Fertigungszwischenschritt, und
- Fig. 3: einen Axialschnitt analog Fig. 1 einer Elektrodenvorrichtung in einer zweiten Ausführungsform.

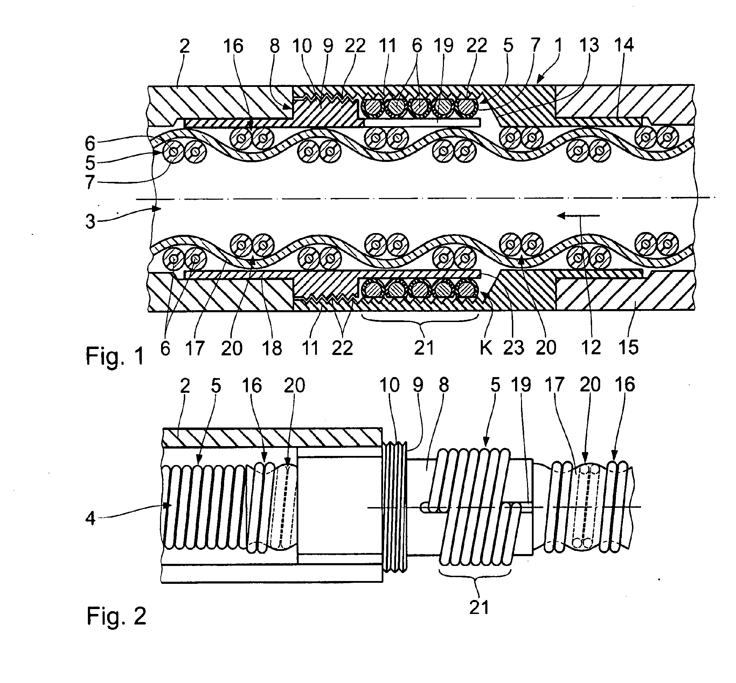

Fig. 1 und 2 zeigt ausschnittsweise einen Herzelektrodenkatheter im Bereich einer vor dem distalen Ende angeordneten Ringelektrode 1, die isodiametrisch mit dem eigentlichen lang gestreckten, schlauchartigen Elektrodenkörper 2 aus einem körperverträglichen Silikonmaterial ausgebildet ist.
Der Elektrodenkörper 2 nimmt koaxial ein Vierfach-Wendelleitungsbündel 4 auf, das für den elektrischen Anschluss der Ringelektrode 1 und einer nicht dargestellten Tip-Elektrode Sorge trägt.

Jede Leitung 5 dieses Leitungsbündels 4 weist jeweils einen Kern 6 mit einer diesen überziehenden Isolierung 7 beispielsweise in Form einer Beschichtung aus einem isolierenden Lack auf.

Wie aus den Fig. 1 und 2 hervorgeht, wird zur Montage und Kontaktierung der Ringelektrode 1 eine Stützhülse 8 eingesetzt, die radial innerhalb der Ringelektrode 1 und des Elektrodenkörpers 2 angeordnet ist. Diese Stützhülse 8 weist etwa mittig bezogen auf seine Längserstreckung einen vorstehenden Kragen 9 mit einem an dessen Außenseite eingeformten Außengewinde 10 auf, das mit einem entsprechenden Innengewinde 11 an der Innenseite der Ringelektrode 1 kooperiert. Das Innengewinde 11 ist dabei gegenüber dem Außengewinde 10 deutlich länger und geht entgegen der Einschraubrichtung 12 in eine sich radial nach innen erstreckende Anschlagschulter 13 über. Diese reduziert den freien Innendurchmesser der Ringelektrode 1 etwa auf den freien Innendurchmesser der Stützhülse 8. Schließlich setzt sich die Ringelektrode 1 entgegen der Einschraubrichtung 12 noch in eine im Außendurchmesser verringerte Aufstecktülle 14 fort, auf die der zum distalen Ende des Elektrodenkatheters hin verlaufende Teil 15 des Elektrodenkörpers 2 aufgesteckt und geeignet fixiert ist.

Zur Herstellung der Kontaktverbindung K zwischen der Ringelektrode 1 und der Leitung 5 wird aus dem Wendelleitungsbündel 4 ausgehend von einer Position vor der Ringelektrode 1 bis zu dem nicht gezeigten Ende des Wendelleitungsbündels 4 ein Zweifach-Teil-Leitungsbündel 16 "ausgewendelt", also aus dem Bündelverbund herausgelöst. Auf das verbleibende Rest-Leitungsbündel 20 wird eine schlauchförmige Isolierhülle 17 aufgeschoben und vorzugsweise aufgeschrumpft, wonach das Zweifach-Teil-Leitungsbündel 16 wieder entsprechend der Wendelung des Rest-Leitungsbündels 20 von außen auf die Isolierhülle 17 über eine bestimmte Länge so aufgewickelt wird, dass noch mehrere Zentimeter Länge des Teil-Leitungsbündels 16 unaufgewickelt bleiben. Anschließend wird die Stützhülse 8 auf die Anordnung aufgeschoben, bis die Stützhülse 8 mit ihrem in Aufschraubrichtung 12 jenseits des Kragens 9 verlaufenden Einsteckflansch 18 in das Lumen 3 des Elektrodenkörpers 2 bis zum Anschlag des Kragens 9 eingeschoben und dort dicht fixiert wird. Anschließend wird das nicht aufgewickelte Ende des Teil-Leitungsbündels 16 durch den entgegen der Aufschraubrichtung 12 offenen, axialparallelen Längsschlitz 19 in der Stützhülse 8 hindurch von innen nach außen geführt und auf der Stützhülse 8 - wie in Fig. 1 und 2 gezeigt - dicht aufgewickelt. Die noch verbleibende Länge des Teil-Leitungsbündels 16 wird anschließend wieder über den Längsschlitz 19 zurück nach innen geführt und auf das Rest-Leitungsbündel 20 mit Isolierhülle 17 aufgewickelt. Die Enden werden dann in geeigneter (nicht dargestellter) Weise elektrisch isoliert und mechanisch im Elektrodenkörper 15 verwahrt.

Ausgehend von dieser in Fig. 2 gezeigten Zwischenmontagestellung wird dann die Ringelektrode 1 mit ihrer Öffnung auf das aus Teil- 16 und Rest-Leitunsgbündel 20 mit Isolierhülle 17 bestehende Leitungsbündel und die Stützhülse 8 mit dem darauf aufgewickelten, zu kontaktierenden Leitungsabschnitt 21 aufgeschoben und mit Hilfe der Gewinde 10, 11 verschraubt. Die Gewinde 10, 11 sind dabei in ihrem Durchmesser so bemessen, dass die Gewindestege 22 des Innengewindes 11 beim Aufstecken und -schrauben der Ringelektrode 1 in die Isolierung 7 der Leitung 5 einschneiden, diese durchdringen und einen elektrischen Kontakt mit dem Kern 6 herstellen. Insoweit ist also kein Abisolierschritt vor dem Aufschrauben der Ringelektrode 1 im Bereich des zu kontaktierenden Leitungsabschnittes 21 notwendig. Die Einschraubtiefe der Ringelektrode 1 wird durch die Anschlagschulter 13 der Ringelektrode 1 begrenzt, die mit der Stirnkante 23 der Stützhülse 8 als Gegenanschlag kooperiert.

Nach dem Aufschrauben des Elektrodenkörpers 2 wird der verbleibende Teil 15 des Elektrodenkörpers 2 - wie beschrieben - auf das Leitungsbündel 4 aufgeschoben und mit der Ringelektrode 2 über die Aufstecktülle 14 dauerhaft verbunden.

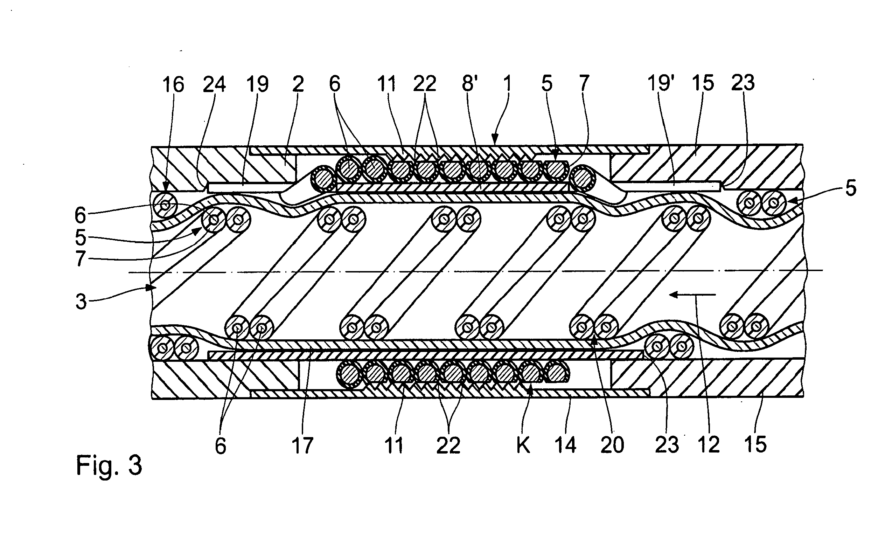

Bei dem in Fig. 3 gezeigten Ausführungsbeispiel ist die Gewindeverbindung zwischen Ringelektrode 1 und Stützhülse 8' nicht vorhanden. Letztere weist lediglich zwei zu den jeweils einander abgewandten Stirnkanten 23, 24 hin offene Längsschlitze 19, 19' auf, über die wiederum das ausgewendelte Teil-Leitungsbündel 16 herausgeführt, auf den zentralen Teil der Stützhülse 8' aufgewickelt und anschließend wieder zum Rest-Leitungsbündel 20 hin nach innen zurückgeführt ist. Danach wird die Ringelektrode 1 mit ihrem Innengewinde 11 auf den auf der Stützhülse 8' außen sitzenden Leitungsabschnitt 21 aufgeschraubt, wobei wiederum der oben erörterte Durchschneideffekt durch die Isolierung 7 der Leitung 5 auftritt und eine elektrische Verbindung zwischen der Ringelektrode 1 und dem Kern 6 der Leitung hergestellt wird. Im Übrigen stimmt die Konstruktion mit dem Ausführungsbeispiel gemäß den Fig. 1 und 2 überein und bedarf keiner nochmaligen Erörterung.

## Patentansprüche

1. Medizinische Elektrodenvorrichtung, insbesondere implantierbare kardiologische Elektrodenvorrichtung, umfassend
- einen lang gestreckten, schlauchartigen Elektrodenkörper (2, 15) aus einem Isoliermaterial,
- eine rotationssymmetrische, insbesondere ringförmige Elektrode (1) am Elektrodenkörper (2, 15),
- eine durch den Elektrodenkörper (2, 15) verlaufende elektrische Leitung (4, 5) mit einem elektrisch leitenden Kern (6) und einer Isolierung (7), wobei der Kern (6) mit der Elektrode (1) elektrisch kontaktverbunden ist,
**gekennzeichnet durch**
- eine rein mechanische Kontaktverbindung (K) zwischen Elektrode (1) und Leitung (5), die aufweist
= eine Stützhülse (8, 8') im Elektrodenkörper (2, 15), auf der der zu kontaktierende Leitungsabschnitt (21) der Leitung (5) in nicht abisoliertem Zustand aufgewickelt ist und auf der die Elektrode (1) unter Umfassen dieses Leitungsabschnitts (21) sitzt, und
= eine Schneidzone (11) an der Innenseite der Elektrode (1), mit der diese unter Durchbrechen der Leitungs-Isolierung (7) mit dem Kern (6) der Leitung (5) in elektrischem Kontakt steht.

2. Elektrodenvorrichtung nach Anspruch 1, **dadurch gekennzeichnet, dass** die Elektrode (1) auf ihrer Innenseite mit einem Innengewinde (11) als Schneidzone versehen ist, mit dem sie auf den zu kontaktierenden Leitungsabschnitt (21) aufgeschraubt ist.

3. Elektrodenvorrichtung nach Anspruch 1 oder 2, **dadurch gekennzeichnet, dass** die Stützhülse (8) mit einem Außengewinde (10) versehen ist, das mit dem Innengewinde (11) der Elektrode (1) zum Verschrauben dieser beiden Teile (1, 8) bei gleichzeitigem Herstellen der Kontaktverbindung (K) zwischen Elektrode (1) und Kern (6) des Leitungsabschnitts (21) korrespondiert.

4. Elektrodenvorrichtung nach Anspruch 3, **dadurch gekennzeichnet, dass** Stützhülse (8) und Elektrode (1) mit Anschlägen (13, 23) zur Begrenzung der Einschraubtiefe zwischen diesen beiden Teilen (1, 8) versehen sind.

5. Elektrodenvorrichtung nach einem der vorgenannten Ansprüche mit einer wendelförmigen Mehrfachleitungsbündel (4), **dadurch gekennzeichnet, dass** die Stützhülse (8, 8') auf dem Mehrfachleitungsbündel (4) angeordnet ist, wobei die Stützhülse (8, 8') mit mindestens einem Längsschlitz (19, 19') zum Herausführen des zu kontaktierenden Leitungsabschnitts (21) des Leitungsbündels (4) zum Aufwickeln auf die Stützhülse (8, 8') versehen ist.

6. Elektrodenvorrichtung nach Anspruch 5, **dadurch gekennzeichnet, dass** die herausgeführte Leitung (16) nach dem kontaktierten Leitungsabschnitt (21) vorzugsweise durch den mindestens einen Längsschlitz (19, 19') auf das Rest-Leitungsbündel (20) zurückgeführt und darauf weitergewickelt ist.

7. Elektrodenvorrichtung nach Anspruch 6, **dadurch gekennzeichnet, dass** zwischen dem aus dem Mehrfachleitungsbündel (4) ausgewendelten Teil-Leitungsbündel (16) und der Stützhülse (8, 8') einerseits und dem verbleibenden Rest-Leitungsbündel (20) andrerseits eine Isolierlage (17) zwischengesetzt ist.

## Claims

1. A medical electrode device, in particular an implantable cardiac electrode device, comprising
- an elongated, tube-shaped electrode body (2, 15) made of an insulating material,
- a rotation-symmetrical, in particular annular electrode (1) on the electrode body (2, 15),
- an electric line (4, 5) which runs through the electrode body (2, 15) and has an electrically conductive core (6) and insulation (7), the core (6) having an electric contact connection with the electrode (1),
**characterized by**
- a purely mechanical contact connection (K) between the electrode (1) and line (5), comprising
= a supporting sleeve (8, 8') in the electrode body (2, 15), on which the line section (21) of the line (5) to be connected is wound in the non-stripped state and on which the electrode (1) sits and envelopes this line section (21), and = a cutting zone (11) on the inside of the electrode (1), with which it is in electric contact by breaking away the line insulation (7) with the core (6) of the line (5).

2. The electrode device according to claim 1, **characterized in that** the electrode (1) on the inside thereof is provided with an internal thread (11) as a cutting zone, with which it is screwed onto the line section (21) to be contacted.

3. The electrode device according to claim 1 or 2, **characterized in that** the supporting sleeve (8) is provided with an external thread (10), which corresponds to the internal thread (11) of the electrode (1) in order to screw these two parts (1, 8) together and at the same time produce the contact connection (K) between the electrode (1) and the core (6) of the line section (21).

4. The electrode device according to claim 3, **characterized in that** the supporting sleeve (8) and electrode (1) are provided with stops (13, 23) to limit the screw-in depth between these two parts (1, 8).

5. An electrode device according to any one of the preceding claims, having a coiled multiline bundle (4), **characterized in that** the supporting sleeve (8, 8') is disposed on the multiline bundle (4), wherein the supporting sleeve (8, 8') is provided with at least one longitudinal slot (19, 19') in order to guide out the line section (21) of the line bundle (4) to be contacted so as to wind it onto the supporting sleeve (8, 8').

6. The electrode device according to claim 5, **characterized in that**, after the contacted line section (21), the line (16) that is guided out is guided back onto the remaining line bundle (20), preferably through the at least one longitudinal slot (19, 19') and wound thereon.

7. The electrode device according to claim 6, **characterized in that** an insulating layer (17) is interposed between the partial line bundle (16) decoiled from the multiline bundle (4) and the supporting sleeve (8, 8'), on the one hand, and the remaining line bundle (20), on the other hand.

## Revendications

1. Dispositif d'électrode médicale, en particulier un dispositif d'électrode cardiaque implantable, comprenant
- un corps d'électrode oblong (2, 15) en forme de tube, constitué d'un matériau isolant,
- une électrode à symétrie de rotation, en particulier annulaire (1), sur le corps d'électrode,
- une ligne électrique (4, 5) traversant le corps d'électrode (2, 15) et possédant un noyau électriquement conducteur (6) ainsi qu'une isolation (7), le noyau (6) possédant une connexion à contact électrique avec l'électrode (1),
**caractérisé par**
- une connexion à contact purement mécanique (K) entre l'électrode (1) et la ligne (5), comprenant
= un manchon de support (8, 8') dans le corps électrique (2, 15), sur lequel la section de ligne (21) de la ligne (5) à contacter est enroulée à l'état non dénudé, et sur lequel s'appuie l'électrode (1) enveloppant cette section de ligne (21) est enveloppée, et
= une zone de coupe (11) à l'intérieur de l'électrode (1), avec laquelle elle est en contact électrique par interruption de l'isolation de ligne (7) avec le noyau (6) de la ligne (5).

2. Dispositif d'électrode selon la revendication 1, **caractérisé en ce que** l'électrode (1) à l'intérieur de celui-ci est pourvue d'un filetage interne (11) comme zone de coupe, avec lequel elle est vissée sur la section de ligne (21) à mettre en contact.

3. Dispositif d'électrode selon la revendication 1 ou 2, **caractérisé en ce que** le manchon de support (8) est pourvu d'un filetage externe (10) correspondant au filetage interne (11) de l'électrode, permettant de visser ces deux parties (1, 8) ensemble, tout en établissant simultanément la connexion à contact (K) entre l'électrode (1) et le noyau (6) de la section de ligne (21).

4. Dispositif d'électrode selon la revendication 3, **caractérisé en ce que** le manchon de support (8) et l'électrode (1) sont pourvus de crans d'arrêt (13, 23) permettant de limiter la profondeur de vissage entre ces deux parties (1, 8).

5. Dispositif d'électrode selon l'une quelconque des revendications précédentes, possédant un faisceau multifilaire spiralé (4), **caractérisé en ce que** le manchon de support (8, 8') est disposé sur le faisceau multifilaire (4), dans lequel le manchon de support (8, 8') est pourvu d'au moins une fente longitudinale (19, 19') permettant de guider vers l'extérieur la section de ligne (21) du faisceau de lignes (4) à mettre en contact, de manière à l'enrouler sur le manchon de support (8, 8').

6. Dispositif d'électrode selon la revendication 5, **caractérisé en ce qu'**après la mise en contact de la section de ligne (21), la ligne (16) guidée vers l'extérieur est ramenée vers le reste du faisceau de lignes (20), de préférence à travers l'au moins une fente longitudinale (19, 19'), et enroulée sur celui-ci.

7. Dispositif d'électrode selon la revendication 6, **caractérisé en ce qu'**une couche isolante (17) est intercalée entre le faisceau de ligne partiel (16) déroulé du faisceau multifilaire (4) et le manchon de support (8, 8') d'une part, et le reste du faisceau de lignes (20) d'autre part.
